# EUROPÄISCHE PATENTANMELDUNG

(11) **EP 1 460 078 A1**
(43) Veröffentlichungstag der Anmeldung: **22.09.2004**
(21) Anmeldenummer: 04005077.5
(22) Anmeldetag: 04.03.2004
(51) Int. Cl.: C07D 495/04, C07D 495/20, C08G 61/12

(54) **3,4-Alkylendioxythiophendiole als Bausteine für elektrisch Leitfähige Polymere zur Verwendung in Kondensatoren**

(30) Priorität: 17.03.2003 DE 10311561
(71) Anmelder: H.C. Starck GmbH, 38642 Goslar (DE)
(72) Erfinder: Wehrmann, Rolf, Dr., 47800 Krefeld (DE); Heuer, Helmut-Werner, Dr., 47829 Krefeld (DE)
(74) Vertreter: Zobel, Manfred, Dr.

(57) **Zusammenfassung**

3,4-Alkylendioxythiophen-Derivate der Formeln I und II 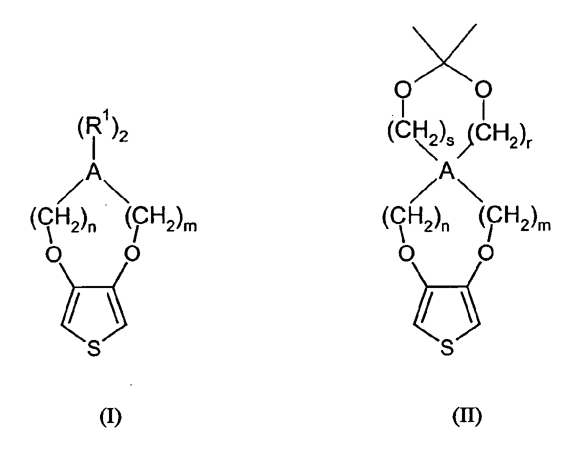 wobei
- n und m: für eine ganze Zahl stehen,
deren Herstellung und die Verwendung zur Herstellung von elektrisch leitfähigen Oligo- oder Polymeren, insbesondere zur Herstellung von Feststoffelektrolyten für Elektrolytkondensatoren, sowie Oligo- oder Polymere, die diese 3,4-Alkylendioxythiophen-Derivate als Wiederholungseinheit enthalten.

## Beschreibung

Die Erfindung betrifft 3,4-Alkylendioxythiophen-Derivate, ein Verfahren zu deren Herstellung und deren Verwendung zur Herstellung von elektrisch leitfähigen Oligo- oder Polymeren, insbesondere zur Herstellung von Feststoffelektrolyten für Elektrolytkondensatoren. Des weiteren betrifft die Erfindung Oligo- oder Polymere, die diese Verbindungen als Wiederholungseinheit enthalten.

Organische leitfähige Polymere besitzen ein breites Anwendungsspektrum. Beispielhaft sei der Einsatz zur Herstellung von Kunststoffbatterien, von Dioden oder Transistoren oder von Solarzellen genannt. Als organische leitfähige Polymere finden beispielsweise Systeme auf Basis von Polyacetylen, Poly(p-phenylen), Polythiophen oder Polypyrrol Anwendung. Aus EP 340 512 B1 ist der Einsatz organischer leitfähiger Polymere als Feststoffelektrolyt für Elektrolytkondensatoren bekannt.

Es sind einige elektrisch leitfähige Oligo- bzw. Polymere bekannt, die aus Thiophenderivaten hergestellt werden. Ein besonderes Beispiel ist das Poly[3,4-ethylendioxythiophen] (PEDT), das insbesondere in der kationischen Form mit Polystyrolsulfonsäure (PSS) als anionischer Komponente eingesetzt wird. PEDT-PSS ist unter der Bezeichnung Baytron® P kommerziell erhältlich.

In EP 340 512 B1 wird die Herstellung eines Feststoffelektrolyten aus 3,4-Ethylen-1,2-dioxythiophen und die Verwendung seines durch oxidative Polymerisation hergestellten, kationischen Polymeren (PEDT) als Feststoffelektrolyt in Elektrolytkondensatoren beschrieben. PEDT als Ersatz von Mangandioxid oder von Ladungstransferkomplexen in Feststoffelektrolytkondensatoren senkt aufgrund der höheren elektrischen Leitfähigkeit den äquivalenten Serienwiderstand des Kondensators und verbessert das Frequenzverhalten.

Der Reststrom eines solchen Kondensators hängt wesentlich von der Qualität des Polymerfilms ab: Dringt Graphit oder Silber durch den Polymerfilm und kommt dadurch in Kontakt mit dem Dielektrikum, so steigt der Reststrom drastisch an, da defekte Stellen in der Oxidschicht nicht mehr über die lokale Zerstörung des leitfähigen Polymers abgekapselt werden können (Selbstheilungseffekt).

Nach einer chemischen Polymerisation müssen gegebenenfalls die Salze, d.h. überschüssiges Oxidationsmittel sowie dessen reduzierte Form, herausgewaschen werden, um Schichten geeigneter Qualität zu erzielen. Andernfalls kann die Kristallisation der Salze im Laufe der Zeit zu einem erhöhten Serienwiderstand durch auftretende Kontaktwiderstände führen. Zudem können die Kristalle bei mechanischer Beanspruchung des Kondensators das Dielektrikum oder die äußeren Kontaktschichten beschädigen, so dass der Reststrom ansteigt. Es ist daher wünschenswert, die Kristallisation von Salzen des Oxidationsmittels sowie Restsalzen dessen reduzierter Form, die trotz Waschung noch im Kondensator verbleiben, zu unterdrücken.

Es bestand daher weiterhin Bedarf, die Leitfähigkeit und Qualität, wie sie für Schichten aus Poly(3,4-ethylendioxythiophen) bekannt ist, insbesondere im Hinblick auf vorangehend beschriebenen Einsatz in Elektrolytkondensatoren zu erhöhen, um eine höhere Leistungsfähigkeit zu erzielen. Dabei ist es vor allem wünschenswert, den äquivalenten Serienwiderstand und den Reststrom von Feststoffelektrolytkondensatoren weiter zu senken. Außerdem ist eine einfache Herstellung der Schichten bzw. der Elektrolytkondensatoren wünschenswert.

Um die Polymereigenschaften, insbesondere die Löslichkeit und Leitfähigkeit des Polymers gezielt auf die jeweiligen Anforderungen einstellen zu können, ist es notwendig, eine Vielzahl geeigneter Monomer-Bausteine zur Verfügung zu haben.

Die Aufgabe bestand daher darin, neue Thiophen-Derivate zur Verfügung zu stellen und Wege zu deren Herstellung aufzufinden. Insbesondere sollten solche Thiophen-Derivate zur Verfügung gestellt werden, welche sich als Feststoffelektrolyte in Elektrolytkondensatoren eignen, wobei Leitfähigkeit und Qualität, insbesondere was die bessere Bindung von Restsalzen und die Homogenität betrifft, im Vergleich zu bekannten Polymeren, wie beispielsweise Poly(3,4-ethylendioxythiophen), verbessert werden sollten.

Es konnten nun neue 3,4-Alkylendioxythiophen-Derivate hergestellt werden, die in der zyklischen Alkyleneinheit zwei Hydroxyalkyl- oder Hydroxy-Gruppen enthalten. Diese Verbindungen zeichnen sich unter anderem dadurch aus, dass die Hydroxyalkyl- oder Hydroxy-Gruppen die Löslichkeit in polaren Lösungsmitteln erhöhen und die Verbindungen durch weitere Umsetzungen an den Hydroxyalkyl- oder Hydroxy-Gruppen weiter derivatisiert werden können. Die elektronische Struktur lässt sich so gezielt beeinflussen, was die Verbindungen als Monomere zur Herstellung leitfähiger Polymere besonders interessant macht. Durch die Gegenwart der Hydroxyalkyl- oder Hydroxy-Gruppen wird weiterhin die Wechselwirkung zu Kondensatormaterialien erhöht.

Gegenstand der Erfindung sind also Verbindungen der Formel I 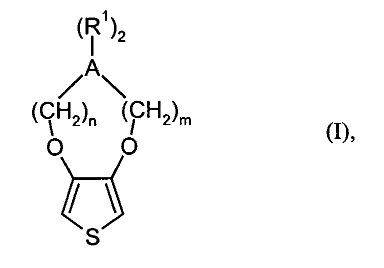 wobei
- n und m: unabhängig voneinander für eine ganze Zahl von 1 bis 5 stehen,
- A: für einen Methylen- oder Ethylenrest steht,
wobei A zwei Substituenten R¹ trägt und
- R¹: jeweils für einen C₁-C₆-Hydroxyalkylrest, bevorzugt einen Hydroxymethyl- oder Hydroxyethylrest, besonders bevorzugt für einen Hydroxymethylrest, oder einen Hydroxylrest steht.

Die beiden Substituenten R¹ können gleich oder verschieden sein, bevorzugt sind sie gleich. Ein Ethylenrest kann dabei die beiden Substituenten R¹ am gleichen oder an verschiedenen C-Atomen, bevorzugt an verschiedenen C-Atomen, tragen.

Bevorzugte erfindungsgemäße Verbindungen sind solche der Formel I-a 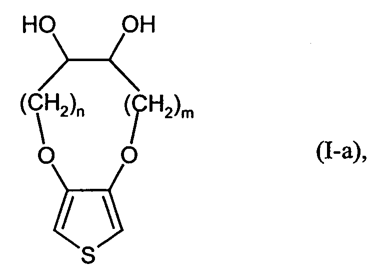 wobei
- n und m: unabhängig voneinander für eine ganze Zahl von 1 bis 5 stehen.

Weiterhin bevorzugte erfindungsgemäße Verbindungen sind solche der Formel I-b 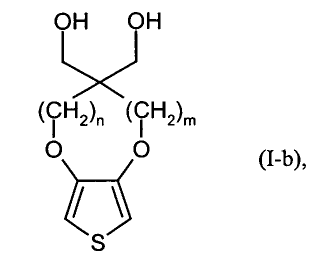 wobei
- n und m: unabhängig voneinander für eine ganze Zahl von 1 bis 5 stehen.

Vorzugsweise stehen n und m unabhängig voneinander für eine ganze Zahl von 1 bis 3, besonders bevorzugt stehen n und m für dieselbe Zahl, insbesondere bevorzugt bedeuten n und m jeweils l.

Es kann für weitere Umsetzungen vorteilhaft sein, wenn die Hydroxyalkyl- oder Hydroxy-Gruppen geschützt werden. Ein bekanntes Vorgehen zum Einführen einer Schutzgruppe für vicinale Diole ist die Umsetzung mit Aceton unter Bildung von 1,3-Dioxolanen. Die Schutzgruppe ist stabil gegenüber Basen und lässt sich durch Behandlung mit Säuren wieder entfernen. Durch die Umsetzung mit Aceton können auch nicht vicinale Dihydroxygruppen, z.B. wie in Verbindungen der Formel I und I-b, geschützt werden.

Gegenstand der Erfindung sind daher weiterhin Verbindungen der Formel II 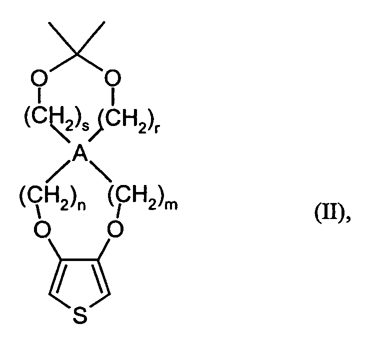 wobei
- r und s: unabhängig voneinander für 0 oder eine ganze Zahl von 1 bis 6 stehen und
- n, m und A: die oben angegebenen Bedeutungen haben.

Bevorzugt stehen r und s unabhängig voneinander für 0 oder 1, besonders bevorzugt stehen r und s für dieselbe Zahl, insbesondere bevorzugt bedeuten r und s jeweils 0 oder 1.

Bevorzugt Gegenstand der Erfindung sind Verbindungen der Formel II-a 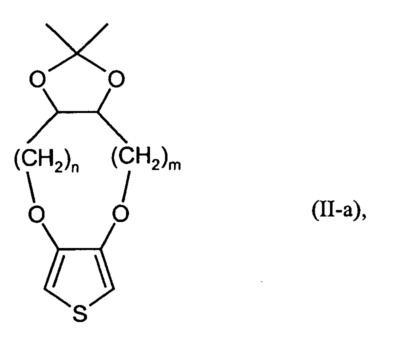 wobei
- n und m: unabhängig voneinander für eine ganze Zahl von 1 bis 5 stehen.

Weiterhin bevorzugt Gegenstand der Erfindung sind Verbindungen der Formel II-b 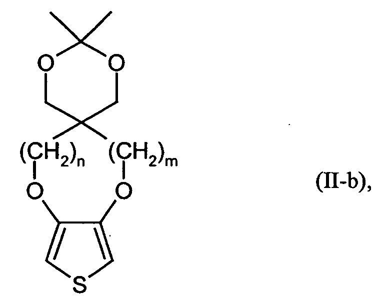 wobei
- n und m: unabhängig voneinander für eine ganze Zahl von 1 bis 5 stehen.

Vorzugsweise stehen n und m unabhängig voneinander für eine ganze Zahl von 1 bis 3, besonders bevorzugt stehen n und m für dieselbe Zahl, insbesondere bevorzugt bedeuten n und m jeweils 1.

Verbindungen der Formel I und II können beispielsweise durch Reaktion geeigneter Thiophene mit α,ω-Tosylat-substituierten Alkylenen hergestellt werden, wobei zur Herstellung von Verbindungen der Formel II anschließend die Schutzgruppe für vicinale Diole, beispielsweise durch Umsetzung mit Aceton, gegebenenfalls erneut eingeführt wird. Unter Verbindungen der Formel I sind hier und im Folgenden auch die bevorzugten Verbindungen der Formel I-a und I-b zu verstehen; unter Verbindungen der Formel II sind hier und im Folgenden auch die bevorzugten Verbindungen der Formel II-a und II-b zu verstehen.

Die Erfindung betrifft daher auch ein Verfahren zur Herstellung einer Verbindung der Formel I oder II, wobei ein Thiophen der Formel III 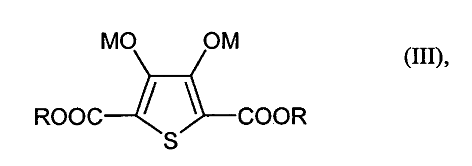 wobei
- R: für C₁-C₁₈-Alkyl und
- M: für H, Li, Na oder K steht,
mit einer Verbindung der Formel IV 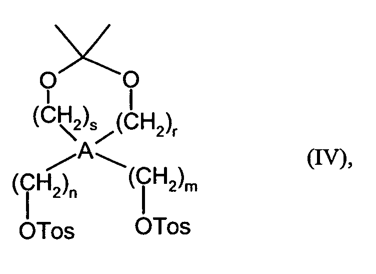 wobei
- n, m, r, s und A: die oben angegebenen Bedeutungen haben und Tos für p-Toluolsulfonyl steht,
zu einer Verbindung der Formel V 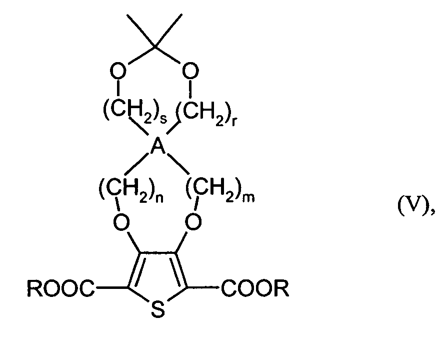 wobei
- R, A, r, s, n und m: die oben angegebenen Bedeutungen haben,
umgesetzt und die Verbindung der Formel V anschließend verseift, angesäuert und decarboxyliert wird.

Bevorzugt ist das erfindungsgemäße Verfahren ein solches, bei dem ein Thiophen der Formel III mit einer Verbindung der Formel IV-a 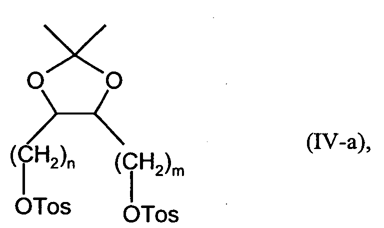 wobei
- n und m: unabhängig voneinander für eine ganze Zahl von 1 bis 5 und Tos für p-Toluolsulfonyl steht,
zu einer Verbindung der Formel V-a 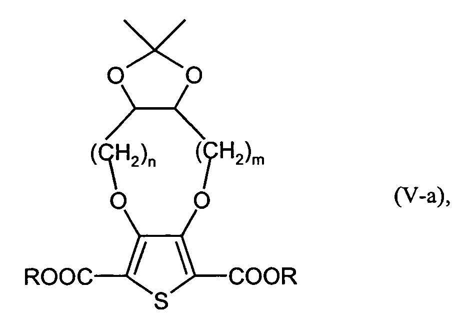 wobei
- R, n und m: die oben angegebenen Bedeutungen haben,
umgesetzt und die Verbindung der Formel V-a anschließend verseift, angesäuert und decarboxyliert wird.

Weiterhin bevorzugt ist das erfindungsgemäße Verfahren ein solches, bei dem ein Thiophen der Formel III mit einer Verbindung der Formel IV-b 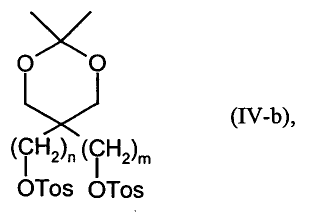 wobei
- n und m: unabhängig voneinander für eine ganze Zahl von 1 bis 5 und Tos für p-Toluolsulfonyl steht,
zu einer Verbindung der Formel V-b 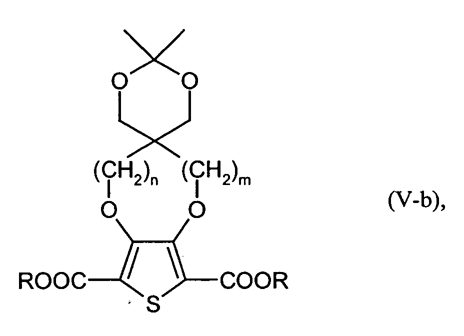 wobei
- R, n und m: die oben angegebenen Bedeutungen haben,
umgesetzt und die Verbindung der Formel V-b anschließend verseift, angesäuert und decarboxyliert wird.

Vorzugsweise steht R in den Formeln III und V für C₁-C₆-Alkyl, beispielsweise Ethyl oder Methyl, insbesondere bevorzugt für Methyl. Unter Verbindungen der Formel V sind hier und im Folgenden auch die bevorzugten Verbindungen der Formel V-a und V-b zu verstehen.

Die Umsetzung des Thiophens der Formel III mit der Verbindung der Formel IV kann beispielsweise bei Normaldruck unter Schutzgasatmosphäre (Ar, N₂) in dipolaren, aprotischen Lösemitteln in Gegenwart einer Base wie z.B. Kaliumcarbonat durchgeführt werden. Unter Verbindungen der Formel IV sind hier und im Folgenden auch die bevorzugten Verbindungen der Formel IV-a und IV-b zu verstehen.

Geeignete Lösungsmittel sind beispielsweise N-Methyl-2-pyrrolidon (NMP), Dimethylformamid, Dimethylacetamid, Dimethylsulfoxid oder hochsiedende Ketone. Bevorzugt wird als Lösungsmittel N-Methyl-2-pyrrolidon eingesetzt.

Die Umsetzung kann beispielsweise bei einer Temperatur von 80 bis 160°C, bevorzugt 90 bis 120°C durchgeführt werden.

Das Ansäuern kann beispielsweise durch Zugabe von Säuren, insbesondere von Essigsäure bei Temperaturen von 10 bis 50°C erfolgen. Vorzugsweise wird soviel Säure zugegeben, dass sich bei der Temperatur der Umsetzung ein pH-Wert von 1 bis 5 einstellt. Hierbei kann bevorzugt gezielt die Schutzgruppe für die Dihydroxygruppierung entfernt werden.

Die Verseifung kann unter allgemein üblichen Bedingungen für eine solche Reaktion durchgeführt werden. Beispielsweise kann dabei so vorgegangen werden, dass die Verbindung der Formel V in verdünnter Natron- oder Kalilauge erhitzt und anschließend mit Salz- oder Schwefelsäure neutralisiert wird. Ein entsprechendes Vorgehen ist beispielsweise bereits aus US-A 5,111,327 bekannt. Mittels der Neutralisierung mit Salz- oder Schwefelsäure können bevorzugt gezielt die Estergruppen entfernt und die freie Dicarbonsäure erhalten werden.

Auch die Decarboxylierung kann auf an und für sich bekannte Weise durchgeführt werden (US-A 5,111,327 und EP 339 340 B1). Beispielsweise wird die Verbindung der Formel V nach der Verseifung und dem Ansäuern in Ethanolamin auf hohe Temperaturen, z. B. 160 bis 200°C, oder in einem dipolaren aprotischen Lösemittel wie Dimethylacetamid oder Dimethylsulfoxid in Gegenwart eines Katalysators wie basischem Kupfercarbonat oder Kupferchromit/Chinolin erhitzt.

Anschließend kann dann gegebenenfalls die Schutzgruppe für vicinale Diole nach bekannten Verfahren, wie z.B. durch Umsetzung mit Aceton unter Bildung von 1,3-Dioxolanen, erneut eingeführt werden.

Durch Umsetzung der Verbindungen der Formel III, worin M und R die oben genannte Bedeutung haben, mit Verbindungen der Formel IV, worin r, s, A, m, n und Tos die oben genannte Bedeutung haben, können zunächst auch gezielt die Verbindungen der Formel V hergestellt werden, worin r, s, A, n, m und R die oben genannte Bedeutung haben. Die Umsetzung kann unter den oben aufgeführten Bedingungen erfolgen. Die gezielte Herstellung der Verbindungen der Formel V kann insofern von Vorteil sein, als dass eine Vorreinigung erfolgen kann. So können in bevorzugten Ausfiihrungsformen durch nachfolgendes Verseifen, Ansäuern und Decarboxylieren die Verbindungen der Formel I in besonders hoher Reinheit erhalten werden. Weiterhin weisen die Verbindungen der Formel V gegebenenfalls eine höhere Lagerstabilität auf als die Verbindungen der Formel I.

Die Verbindungen der Formel I und II lassen sich zur Herstellung von elektrisch leitfähigen Oligound Polymeren verwenden. Dabei kann sowohl nur eine Verbindung der Formel I oder II als Monomer eingesetzt werden, als auch ein Gemisch verschiedener Verbindungen, die unter die Definition der Formeln I und II fallen. Es ist überdies möglich, neben einer oder mehrerer Verbindungen der Formeln I und II auch weitere Thiophen-Derivate als Monomere zuzusetzen, insbesondere 3,4-Ethylendioxythiophen, welches unter dem Handelsnamen Baytron® M kommerziell erhältlich ist.

Die Polymerisation erfolgt entsprechend dem Vorgehen bei der Polymerisation bekannter Thiophen-Derivate. Sie kann beispielsweise oxidativ mit Oxidationsmitteln wie Eisen-III-chlorid oder anderen Eisen-III-Salzen, H₂O₂, Natrium- oder Kaliumperoxodisulfat, Kaliumdichromat, Kaliumpermanganat oder elektrochemisch erfolgen.

Die Erfindung betrifft daher weiterhin die Verwendung von Verbindungen der Formeln I und II zur Herstellung von elektrisch leitfähigen Oligo- und Polymeren und elektrisch leitfähige Oligound Polymere, die durch Polymerisation einer Verbindung der Formel I und/oder II hergestellt werden.

Die Erfindung betrifft insbesondere elektrisch leitfähige Oligo- und Polymere, die als Wiederholungseinheiten Struktureinheiten der Formel VI 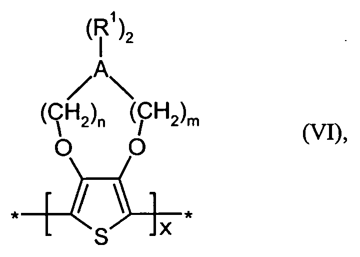 wobei
- A und R¹: die oben angegebenen Bedeutungen haben und
- n und m: jeweils unabhängig voneinander für eine ganze Zahl von 1 bis 5 und x für eine ganze Zahl von 2 bis 10000 stehen,
und/oder der Formel VII, 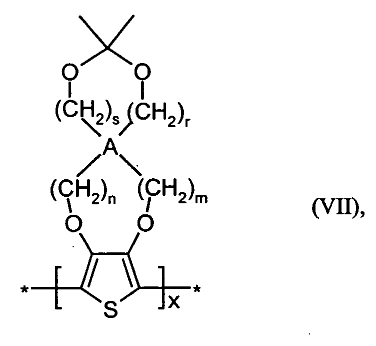 wobei
- n, m, r, s und A: die oben für die Formel II angegebenen Bedeutungen haben und x für eine ganze Zahl von 2 bis 10000 steht,
enthalten.

Bevorzugte erfindungsgemäße elektrisch leitfähige Oligo- und Polymere sind solche, die als Wiederholungseinheiten Struktureinheiten der Formel VI-a und /oder VII-a 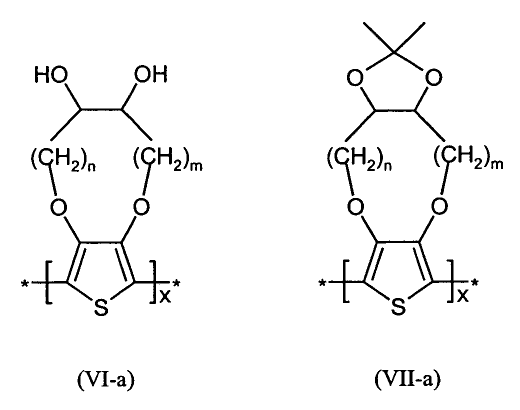 worin n, m und x die oben angegebenen Bedeutungen haben, enthalten.

Weiterhin sind bevorzugte erfindungsgemäße elektrisch leitfähige Oligo- und Polymere solche, die als Wiederholungseinheiten Struktureinheiten der Formel VI-b und /oder VII-b 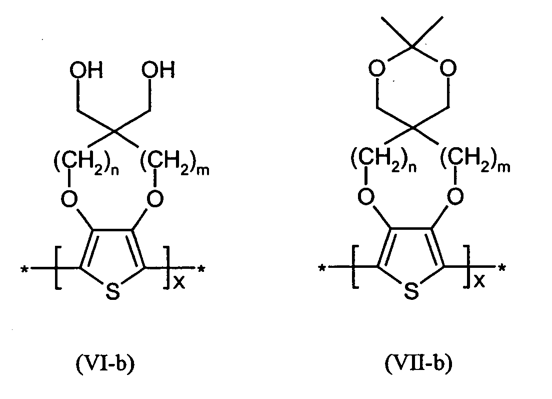 worin n, m und x die oben angegebenen Bedeutungen haben, enthalten.

Bevorzugt steht x für 2 bis 5000, insbesondere bevorzugt für 2 bis 100.

Die Struktureinheiten der Formeln VI, VI-a, VI-b und VII, VII-a, VII-b können, wie in den Formeln dargestellt, neutral sein. Es ist aber auch möglich, dass sie eine positive Ladung tragen. In diesem Fall enthalten die erfindungsgemäßen Polymere Anionen als Gegenionen. Vorzugsweise sind diese Anionen wiederum polymer aufgebaut, insbesondere bevorzugt handelt es sich bei den Polyanionen um Polystyrolsulfonat.

Die erfindungsgemäßen Oligo- oder Polymere können beispielsweise als Lochinjektionsschicht in organischen Leuchtdioden, als Glättungsschicht für ITO-Schichten in organischen Leuchtdioden, als leitfähige Schichten in anorganischen Leuchtdioden, als farbgebende elektrochrome oder ionenspeichernde Gegenelektrode in elektrochromen Anordnungen, in der Antistatik, zur Durchkontaktierung von Leiterplatten, im Korrosionsschutz, in Sensoren oder in organischen Feldeffekt-Transistoren zum Einsatz kommen.

Gegenstand der Erfindung sind auch Mischungen der erfmdungsgemäßen Thiophen-Derivate der Formeln I oder II mit 3,4-Ethylendioxythiophen (EDT).

Diese nachfolgenden Beispiele sind nicht als Einschränkung des allgemeinen Erfindungsgedankens zu verstehen, sondern dienen ausschließlich der weiteren Erläuterung der Erfindung.

### Beispiele

### Beispiel 1

2,46 g (0,0106 mol) 3,4-Dihydroxy-thiophen-1,2-dimethylester (Bayer AG, Leverkusen) und 5 g (0,0106 mol) 1,4-Di-o-Tosyl-2,3-isopropyliden-D-threitol (Fa. Aldrich) wurden gemeinsam mit 3,66 g (0,0265 mol) Kaliumcarbonat in 130 ml getrocknetem N-Methylpyrrolidon (NMP) 18 Stunden bei 100°C gerührt. Der Reaktionsansatz wurde zur Aufarbeitung mit Wasser und Methylenchlorid versetzt und neutralgeschüttelt. Die organische Phase wurde abgetrennt und mit Natriumsulfat getrocknet. Nach dem Einengen der organischen Phase und Trocknung erhielt man 2,2 g eines braunen Rohproduktes, welches das gewünschte Produkt mit der Masse 358 als Hauptkomponente enthielt (Analyse GC-MS).

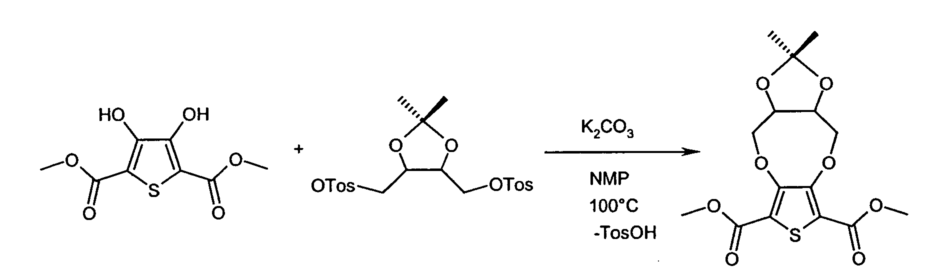

### Beispiel 2

Durch Chromatographie über Siliciumdioxid mit einer Mischung aus Toluol/Ethylacetat (zu Beginn 10 Volumenteile Toluol auf 1 Volumenteil Ethylacetat, dann 5:1, 3:1 und schließlich 1:1) erhielt man nach Abspaltung der Schutzgruppe das gewünschte Diol mit der Masse 318 (Analyse GC-MS nach Trimethylsilyl- Derivatisierung (TMS-Derivatisierung); Di-TMS-MG 462).

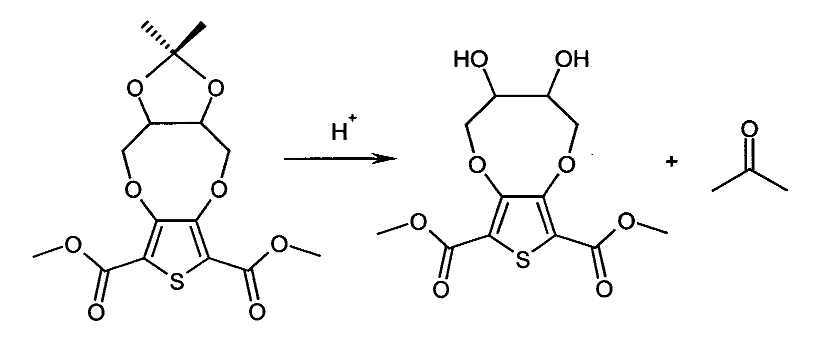

### Beispiel 3

Beispiel 1 wurde wiederholt, wobei die Menge der Edukte vervierfacht wurde. Man erhielt 9,3 g eines dunkelbraunen Rohproduktes, welches nach chromatographischer Reinigung und Spaltung analog Beispiel 2 2,0 g (0,0063 mol, 14,9 % der Theorie) eines gelben kristallinen Feststoffs als Produkt lieferte. Die Freisetzung der freien Thiophenverbindung kann in bekannter Weise durch Esterspaltung (Verseifung und Ansäuern) und Decarboxylierung, beispielsweise analog US-A 5,111,327 und EP 339 340 B1 erfolgen.

### Beispiel 4

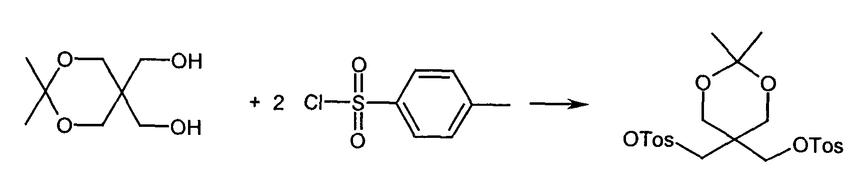

10 g (56,8 mmol) des einseitig ketalisierten Pentaerythrits werden in 24,2 g (150 mmol) Triethylamin vorgelegt. Zu dieser Lösung werden Raumtemperatur 22,9 g (120 mmol) p-Toluolsulfonsäurechlorid in 50 ml Methylenchlorid getropft. Es erfolgt eine leicht exotherme Reaktion unter Trübung der Lösung. Man lässt ca. 3,5 Stunden bei Raumtemperatur und anschließend 3 Stunden bei 40 - 45°C nachrühren.

Der gesamte Ansatz wird am Rotationsverdampfer zur Trockne eingelegt und der Rückstand dreimal mit 200 ml Methanol gewaschen. Die methanolische Lösung wird dekantiert und der weiße Rückstand im Wasserstrahlvakuum getrocknet.

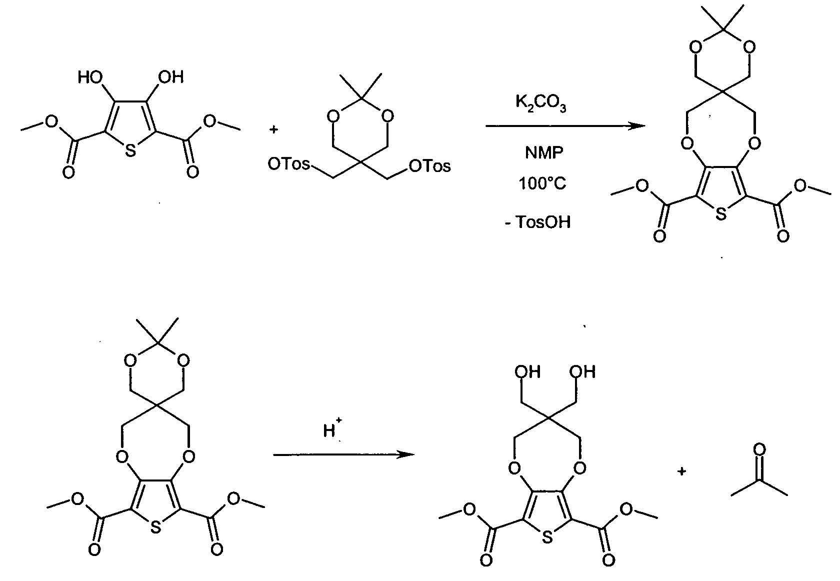

2,55 g (0,011 mol) 3,4-Dihydroxy-thiophen-1,2-dimethylester und 5 g (0,011 mol) des Ditosylats des halbseitig geschützten Pentaerythrits werden mit 3,8 g (0,028 mol) Kaliumcarbonat in 100 ml N-Methylpyrrolidon 18 Stunden bei 100°C gerührt. Der Reaktionsansatz wird zur Aufarbeitung mit Wasser und Methylenchlorid versetzt und neutral gewaschen. Die organische Phase wird abgetrennt und mit Natriumsulfat getrocknet. Nach dem Einengen der organischen Phase und Trocknung erhielt man 2,8 g Produkt.

Analyse: GC-MS zeigt das gewünschte Molekül als mit der Masse 372 als Hauptprodukt.

Die weitere Feinreinigung erfolgt durch Chromatographie über Siliciumdioxid analog Beispiel 2. Analog Beispiel 3 erfolgt die Abspaltung der Schutzgruppe. Man erhält das gewünschte Produkt (Analyse GC-MS nach Trimethylsilyl-Derivatisierung (TMS-Derivatisierung); Di-TMS-MG 476).

## Patentansprüche

1. Verbindung der Formel I 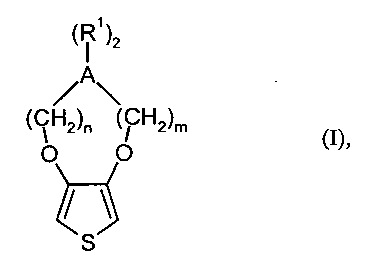 wobei
n und m unabhängig voneinander für eine ganze Zahl von 1 bis 5 stehen,
A für einen Methylen- oder Ethylenrest steht,
wobei A zwei Substituenten R¹ trägt und
R¹ jeweils für einen C₁-C₆-Hydroxyalkylrest, bevorzugt einen Hydroxymethyl- oder Hydroxyethylrest, besonders bevorzugt für einen Hydroxymethylrest, oder einen Hydroxylreste steht.

2. Verbindung gemäß Anspruch 1 der Formel I-a 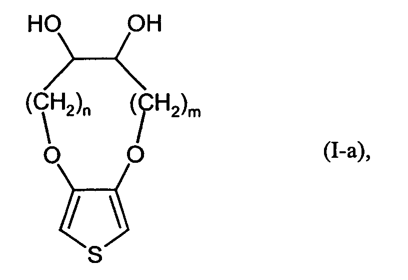 wobei
n und m unabhängig voneinander für eine ganze Zahl von 1 bis 5 stehen.

3. Verbindung gemäß Anspruch 1 der Formel I-b 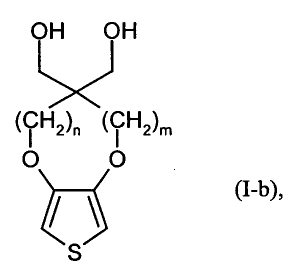 wobei
n und m unabhängig voneinander für eine ganze Zahl von 1 bis 5 stehen.

4. Verbindung gemäß wenigstens einem der Ansprüche 1 bis 3, **dadurch gekennzeichnet, dass** n und m jeweils 1 bedeuten.

5. Verbindung der Formel II 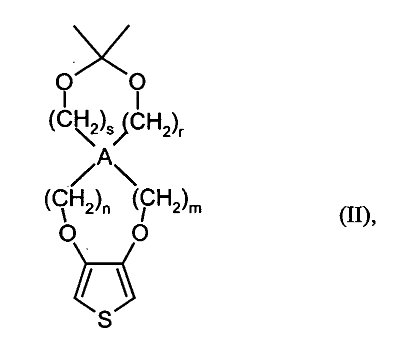 wobei
r und s unabhängig voneinander für 0 oder eine ganze Zahl von 1 bis 6 stehen,
n und m unabhängig voneinander für eine ganze Zahl von 1 bis 5 stehen,
A für einen Methylen- oder Ethylenrest steht.

6. Verbindung gemäß Anspruch 5 der Formel II-a 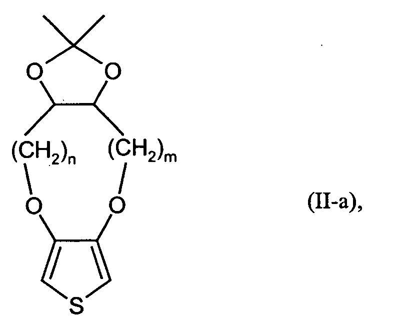 wobei
n und m unabhängig voneinander für eine ganze Zahl von 1 bis 5 stehen.

7. Verbindung gemäß Anspruch 5 der Formel II-b 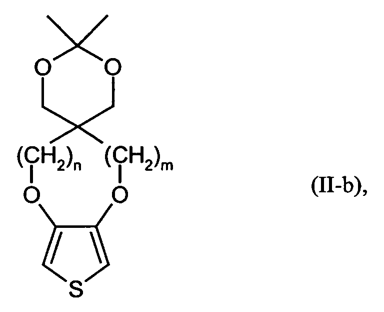 wobei
n und m unabhängig voneinander für eine ganze Zahl von 1 bis 5 stehen.

8. Verbindung gemäß wenigstens einem der Ansprüche 5 bis 7, **dadurch gekennzeichnet, dass** n und m jeweils 1 bedeuten.

9. Verfahren zur Herstellung einer Verbindung gemäß wenigstens eines der Ansprüche 1 bis 4, **dadurch gekennzeichnet, dass** ein Thiophen der Formel III 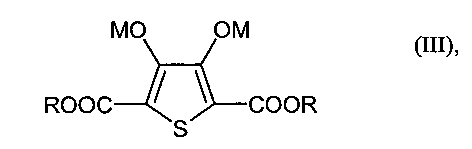 wobei
R für C₁-C₁₈-Alkyl und
M für H, Li, Na oder K steht,
mit einer Verbindung der Formel IV 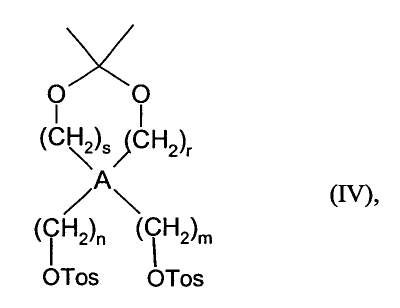 wobei
r und s unabhängig voneinander für 0 oder eine ganze Zahl von 1 bis 6 stehen,
n und m unabhängig voneinander für eine ganze Zahl von 1 bis 5 stehen,
A für einen Methylen- oder Ethylenrest steht,
Tos für p-Toluolsulfonyl steht,
zu einer Verbindung der Formel V 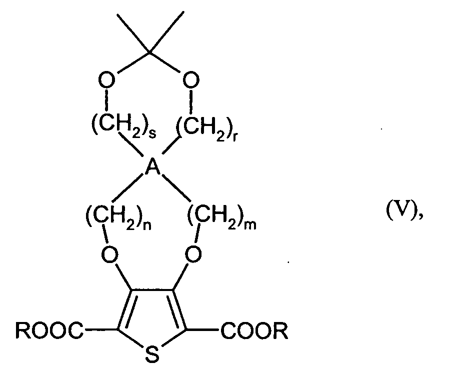 wobei
A, R, r, s, n und m die für die Formeln III und IV angegebenen Bedeutungen haben,
umgesetzt und die Verbindung der Formel V anschließend verseift, angesäuert und decarboxyliert wird.

10. Verfahren gemäß Anspruch 9, **dadurch gekennzeichnet, dass** ein Thiophen der Formel III 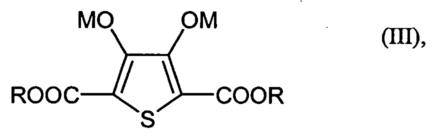 wobei
M und R die in Anspruch 9 angegebenen Bedeutungen haben,
mit einer Verbindung der Formel IV-a 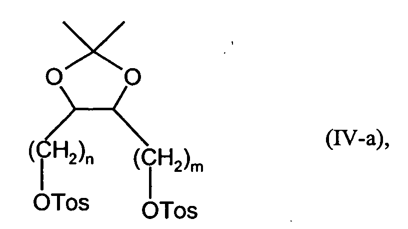 wobei
n und m unabhängig voneinander für eine ganze Zahl von 1 bis 5 stehen und
Tos für p-Toluolsulfonyl steht,
zu einer Verbindung der Formel V-a 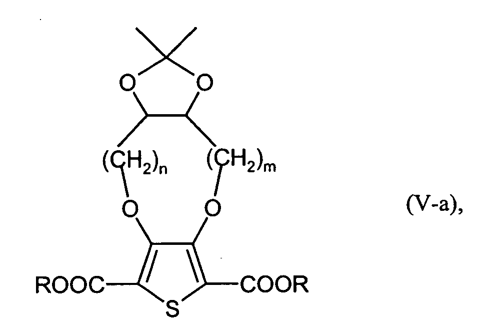 wobei
R, n und m die für die Formeln III und IV-a angegebenen Bedeutungen haben,
umgesetzt und die Verbindung der Formel V-a anschließend verseift, angesäuert und decarboxyliert wird.

11. Verfahren gemäß Anspruch 9, **dadurch gekennzeichnet, dass** ein Thiophen der Formel III 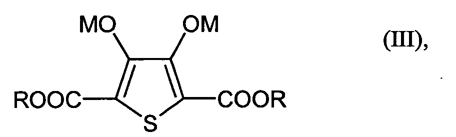 wobei
M und R die in Anspruch 9 angegebenen Bedeutungen haben,
mit einer Verbindung der Formel IV-b 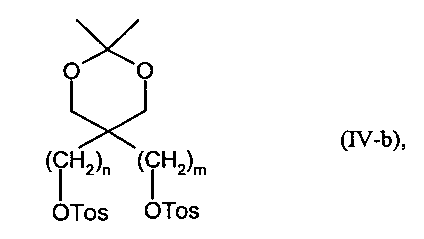 wobei
n und m unabhängig voneinander für eine ganze Zahl von 1 bis 5 und
Tos für p-Toluolsulfonyl steht,
zu einer Verbindung der Formel V-b 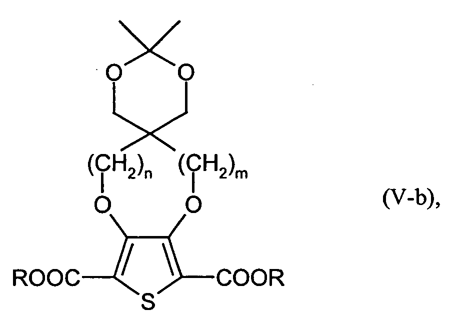 wobei
R, n und m die für die Formeln III und IV-b angegebenen Bedeutungen haben,
umgesetzt und die Verbindung der Formel V-b anschließend verseift, angesäuert und decarboxyliert wird.

12. Verwendung einer Verbindung gemäß wenigstens eines der Ansprüche 1 bis 8 zur Herstellung eines elektrisch leitfähigen Oligo- oder Polymers.

13. Verwendung einer Verbindung gemäß wenigstens eines der Ansprüche 1 bis 8 bei der Kondensatorherstellung.

14. Verwendung einer Mischung enthaltend mindestens eine Verbindung gemäß wenigstens eines der Ansprüche 1 bis 8 und 3,4-Ethylendioxythiophen bei der Kondensatorherstellung.

15. Elektrisch leitfähiges Oligo- oder Polymer enthaltend Struktureinheiten der Formel VI 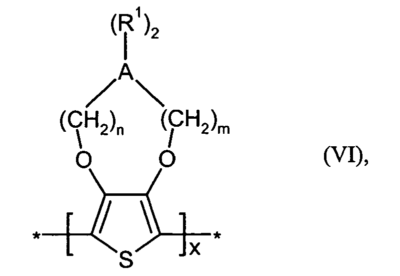 wobei
A für einen Methylen- oder Ethylenrest steht,
wobei A zwei Substituenten R¹ trägt und
R¹ jeweils für einen C₁-C₆-Hydroxyalkylrest, bevorzugt einen Hydroxymethyl- oder Hydroxyethylrest, besonders bevorzugt für einen Hydroxymethylrest, oder einen Hydroxylreste steht,
n und m jeweils unabhängig voneinander für eine ganze Zahl von 1 bis 5 stehen und
x für eine ganze Zahl von 2 bis 10000 steht,
und/oder der Formel VII, 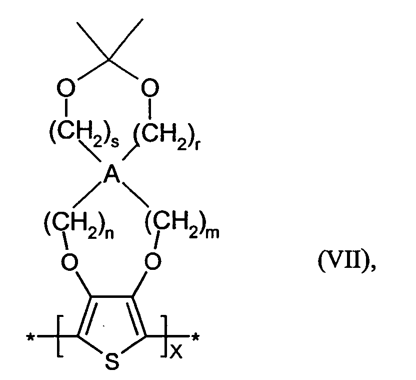 wobei
r und s unabhängig voneinander für 0 oder eine ganze Zahl von 1 bis 6 stehen und
n, m, A und x die für die Formel VI angegebenen Bedeutungen haben.

16. Elektrisch leitfähiges Oligo- oder Polymer gemäß Anspruch 15 enthaltend Struktureinheiten der Formel VI-a 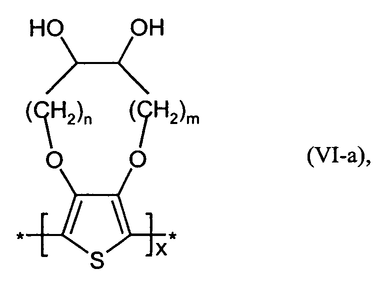 wobei
n, m und x die in Anspruch 15 angegebene Bedeutung haben,
und/oder der Formel VII-a, 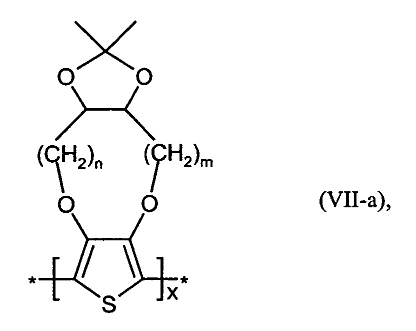 wobei
n, m und x die in Anspruch 15 angegebene Bedeutung haben.

17. Elektrisch leitfähiges Oligo- oder Polymer gemäß Anspruch 15 enthaltend Struktureinheiten der Formel VI-b 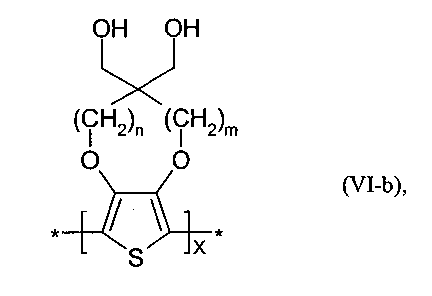 wobei
n, m und x die in Anspruch 15 angegebene Bedeutung haben.
und/oder der Formel VII-b 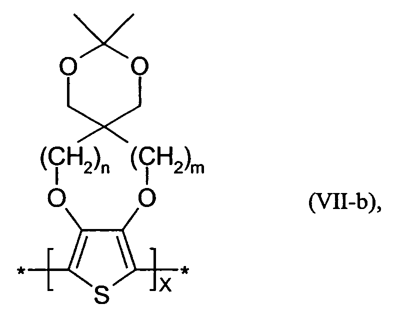 wobei
n, m und x die in Anspruch 15 angegebene Bedeutung haben.

18. Elektrisch leitfähiges Oligo- oder Polymer gemäß wenigstens einem der Ansprüche 15 bis 17, **dadurch gekennzeichnet, dass** es sich dabei um ein Copolymer handelt, das neben Struktureinheiten der Formel VI und/oder VII auch 3,4-Ethylendioxythiophen-Struktureinheiten enthält.

19. Verwendung eines elektrisch leitfähigen Oligo- oder Polymers gemäß wenigstens einem der Ansprüche 15 bis 18 als Lochinjektionsschicht in organischen Leuchtdioden, Glättungsschicht für ITO-Schichten in organischen Leuchtdioden oder als farbgebende elektrochrome oder ionenspeichernde Gegenelektrode in elektrochromen Anordnungen.

20. Verwendung eines elektrisch leitfähigen Oligo- oder Polymers gemäß wenigstens einem der Ansprüche 15 bis 18 in organischen Feldeffekt-Transistoren.

21. Verwendung eines elektrisch leitfähigen Oligo- oder Polymers gemäß wenigstens einem der Ansprüche 15 bis 18 in der Antistatik, zur Durchkontaktierung von Leiterplatten, im Korrosionsschutz, in Sensoren oder als leitfähige Schicht in anorganischen Leuchtdioden.

22. Verbindung der Formel V 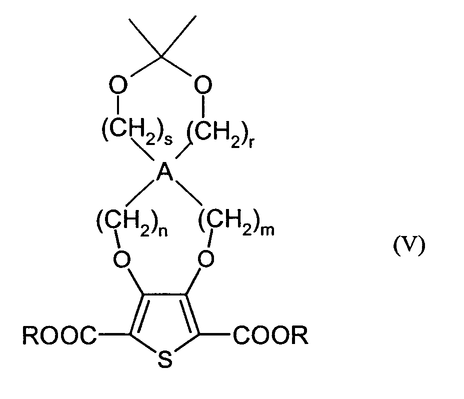 wobei
R für C₁-C₁₈-Alkyl steht,
r und s unabhängig voneinander für 0 oder eine ganze Zahl von 1 bis 6 stehen,
n und m unabhängig voneinander für eine ganze Zahl von 1 bis 5 stehen,
A für einen Methylen- oder Ethylenrest steht.

23. Verbindung gemäß Anspruch 22 der Formel V-a oder V-b, 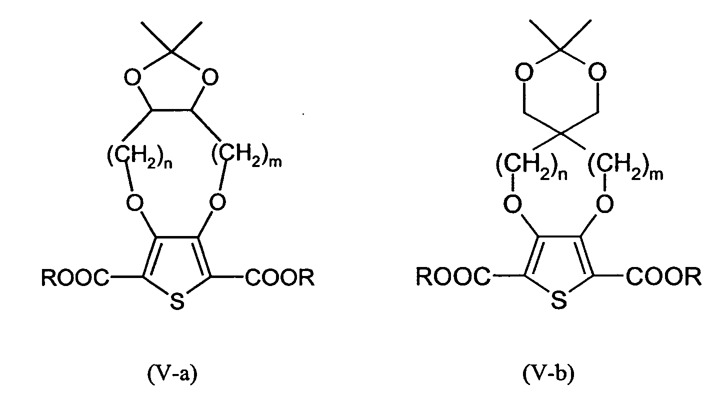 wobei
R, n und m die in Anspruch 22 angegebenen Bedeutungen haben.
